# EUROPÄISCHE PATENTANMELDUNG

(11) **EP 0 533 206 A2**
(43) Veröffentlichungstag der Anmeldung: **24.03.1993**
(21) Anmeldenummer: 92116130.3
(22) Anmeldetag: 21.09.1992
(51) Int. Cl.: A63B 21/00, A61B 5/22

(54) **Vorrichtung zu gymnastischen Zwecken**

(30) Priorität: 20.09.1991 DE 4131422
(71) Anmelder: Cohausz, Helge B., D-40237 Düsseldorf (DE)
(72) Erfinder: Cohausz, Helge B., D-40237 Düsseldorf (DE)

(57) **Zusammenfassung**

Die Erfindung betrifft eine Vorrichtung (1) in Kissenform für gymnastische Zwecke. auf die vom Menschen eine Druckkraft ausübbar ist, wobei durch eine Einrichtung zum Messen und/oder Anzeigen (6,7) des auf die Vorrichtung (1) durch den menschlichen Körper ausgeübten Druckes, wobei die Mess- und/oder Anzeigeeinrichtung (6,7) bei Überschreiten eines bestimmten Druckes ein akustisches und/oder optisches Signal abgibt.

## Beschreibung

Die Erfindung betrifft eine Vorrichtung für gymnastische Zwecke, auf die vom Menschen eine Druckkraft ausübbar ist.

Es sind die verschiedensten Vorrichtungen für gymnastische Zwecke bekannt, bei denen Kraft in Form von Druck ausgeübt wird. In der Mehrzahl werden hierbei Federelemente zusammengedrückt oder auseinandergezogen oder aber Gewichte angehoben, um Muskeln zu erzeugen. Darüberhinaus ist es bekannt, Kraft aufzuwenden, ohne einen Teil stärker zu bewegen. Bei letzteren Vorrichtungen ist dem Benutzer nicht deutlich, wie stark die Druckkraft ist, die er momentan ausübt.

Aufgabe der Erfindung ist es, eine Vorrichtung der eingangs genannten Art so zu verbessern, daß bei einfacher Konstruktion und Bedienung und bei hoher Wirksamkeit der die Vorrichtung benutzende Mensch sofort erfährt, welche Kraft von ihm aufgewendet wird und aufgewendet werden muß.

Diese Aufgabe wird erfindungsgemäß dadurch gelöst, durch eine Einrichtung zum Messen und/oder Anzeigen des auf die Vorrichtung durch den menschlichen Körper ausgeübten Druckes, wobei die Mess- und/oder Anzeigeeinrichtung bei Überschreiten eines bestimmten Druckes ein akustisches und/oder optisches Signal abgibt.

Hierdurch erfährt der Mensch nicht anhand einer oft schwer ablesbaren Skala welche Kraft er aufgewendet hat, sondern bei Erreichen der erforderlichen oder von ihm gewünschten Kraft wird er akustisch und/oder optisch informiert, so daß er von der richtig aufgewendeten Kraft erfährt und stets optimal informiert ist, ohne eine Skala versehentlich verkehrt ablesen zu können. Es wird somit seine Motivation erhöht und eine Fehlbedienung sicher vermieden.

Besonders vorteilhaft ist es hierbei, wenn der bestimmte Druck einstellbar und/oder verstellbar ist.

Vorteilhafte Ausgestaltungen der Erfindung sind in den Unteransprüchen aufgeführt. Hierbei ist von größtem Vorteil, daß eine solche Vorrichtung aufgrund ihrer flachen Bauweise für die verschiedensten gymnastischen Übungen von größtem Vorteil sind. So kann sie auch unter das Rückgrad gelegt werden, um Übungen der Muskeln der Wirbelsäule fehlerfrei und mit hoher Effektivität durchzuführen.

Ausführungsbeispiele der Erfindung sind in den Zeichnungen dargestellt und werden im folgenden näher beschrieben. Es zeigen
- Figur 1: eine perspektivische Darstellung eines ersten Ausführungsbeispiels,

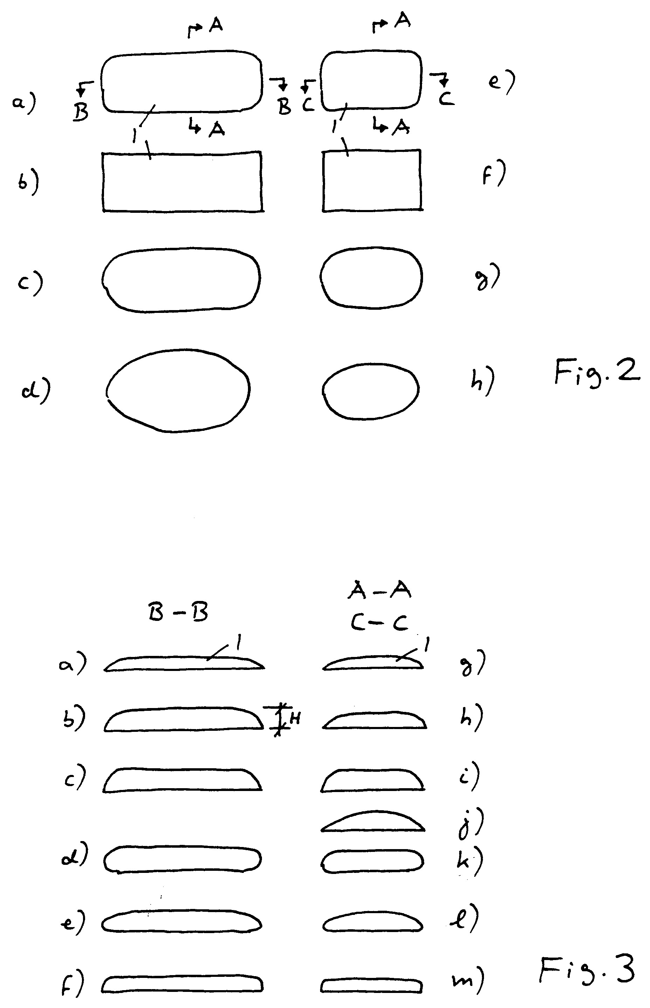

- Figur 2: a bis h Draufsichten verschiedener Ausführungsformen,
- Figur 3: a bis f verschiedene Ausführungsformen im Längsschnitt B-B und
- Figur 4: g bis m verschiedene Ausführungsformen im Querschnitt A-A bzw. Längsschnitt C-C.

Die Vorrichtung hat die Form eines flachen Kissens, das in der Regel waagerecht auf den Boden, senkrecht an einer Wand oder schräg auf eine Schräge gelegt wird. Hierbei kann die Vorrichtung die verschiedensten Formen besitzen, wie dies in den Zeichnungen und in den Ansprüchen 4 bis 10 und 14 und 15 ausgeführt ist. Insbesondere die Oberseite kann eine flächige Polsterung aufweisen, wobei auch die Randbereiche der Oberseite gepolstert sein können, um die Benutzung angenehmer zu machen.

Wird vom Benutzer auf die Vorrichtung von oben, d.h. von der Oberseite der Vorrichtung aus, Kraft auf die Oberseite ausgeübt, so ist die Vorrichtung nicht oder nur wenig zusammendrückbar, d.h. die Polsterung soweit vorhanden gibt etwas nach, aber im übrigen gibt die Vorrichtung nicht oder nur wenige Millimeter nach.

In der Vorrichtung befindet sich eine Druckmeßvorrichtung, insbesondere mit einem piezoelektrischem Teil, das den Druck mißt und einer elektronischen Vorrichtung übergibt, die bei Überschreiten eines bestimmten Druckes ein akustisches und/oder optisches Signal abgibt. Diese Meß- und/oder Anzeigeeinrichtung ist auf einem bestimmten Druck einstellbar und/oder verstellbar, d.h., wenn dieser bestimmte vorgewählte Druck erreicht wird, wird erst das akustische und/oder optische Signal abgegeben. Hierbei kann diese Einrichtung zusätzlich noch so ausgeführt sein, daß sie während des Einhaltens des bestimmten Druckes über eine bestimmte Dauer ständig das Signal abgibt oder aber nach Beendigung des Einhaltens dieses bestimmten Druckes Signal, d.h. entweder dasselbe Signal oder ein anderes Signal abgibt. Hierdurch ist es für den Benutzer möglich, sehr genau einen bestimmten Druck über längere Zeit in gleicher Höhe auszuüben. Besonders vorteilhaft ist es hierbei, wenn die Druckstärke durch die Signalstärke oder Signalhöhe bzw. durch die Art des optischen Signals variiert. Wird also die Vorrichtung weniger stark gedrückt als vorgewählt, so kann z.B. ein tiefer oder unangenehmer Ton oder ein flackerndes Licht erzeugt werden und bei Erreichen des vorgewählten Druckes ein angenehmer Ton oder ein leiserer Ton bzw. eine Melodie oder ein angenehmes Farbsignal. Dies fördert wesentlich die Motivation, das Gerät effektiv und richtig zu benutzen.

Die Funktion der Meß- und Signaleinrichtung ist unabhängig von der Lage der Vorrichtung im Raum, d.h. bei senkrecht oder schräger Lage werden dieselben korrekten Werte erzielt. Damit ist das Gerät vielfältig verwendbar.

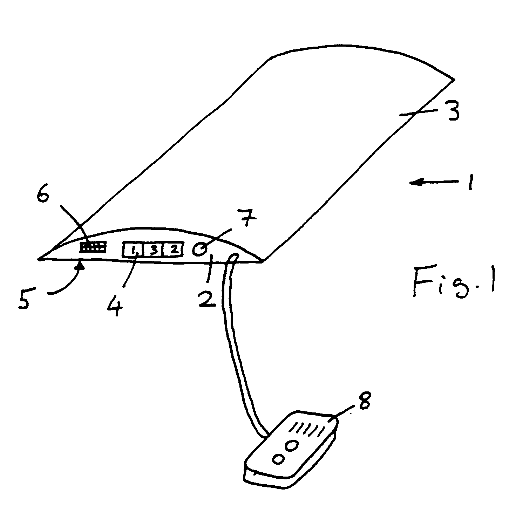

Das Ausführungsbeispiel nach Figur 1 zeigt die Vorrichtung in einer zylinderabschnittförmigen Ausführung mit einer ebenen Unterseite 5, die besonders gut für die Behandlung von Rückenschmerzen geeignet ist. Die Vorrichtung 1 weist an einer der beiden Stirnseiten 2 eine Einstellvorrichtung 4 auf, mit Zahlen, durch die die Kraft eingestellt wird, die durch den Benutzer erzeugt werden soll. Auf der nicht gezeigten gegenüberliegenden Stirnseite oder an derselben Stirnseite kann eine zweite Einstellvorrichtung vorgesehen sein, um die Dauer einzustellen, über die die vorgewählte Kraft eingehalten werden soll. Die Oberseite 3 der Vorrichtung 1 als auch die Ränder sind gepolstert. An der Stirnseite 2 sind auch eine Lampe 7 und ein kleiner Lautsprecher 6 zur Abgabe von Signalen befestigt. Zur Verteilung sind punktförmige Belastungen in oder unter der Oberseite oberhalb der Messeinrichtung mit einer insbesondere gewölbten Fläche aus biegesteifem Material angeordnet, unter der die druckmessenden Einrichtungen befestigt sind.

Es können mehrere den Druck messende Einrichtungen (mechanisch, elektrisch oder mit Piezokristall) in der Vorrichtung angeordnet sein. Die von den Einrichtunegen gemessenen Drücke können in einer angeschlossenen elektrischen Einrichtung (Rechner, Kleincomputer) aufgenommen und bewertet werden, wobei entweder ein Mittel aus den Werten oder der Höchstwert verwendet wird.Im folgenden wird anhand eines Beispiels der Gebrauch der Vorrichtung beschrieben.

Bei Schmerzen der Wirbelsäule in der Lendengegend kann die die Vorrichtung benutzende Person sich mit dem Rücken auf den Boden legen und die Beine anziehen und die Hände unter den Kopf legen. Zuvor hat die Person die Vorrichtung mit der Längsrichtung quer zur Wirbelsäule unter die Taille gelegt. Danach kann die Person mit der Wirbelsäule Druck auf die Vorrichtung in Richtung des Bodens ausüben und hierbei einen sehr hohen Druck nach Möglichkeit auf die Vorrichtung über längere Zeit in gleicher Stärke erzeugen. Dies ist eine optimale Übung, um Rückenschmerzen zu beheben.

Die Außenseite der Erfindung ist von einem Schlauch gebildet. Die Erfindung ist über eine Leitung und/oder Schlauch mit einer Fernbedienung 8 und/oder einer Fernanzeige 8 verbunden. Ferner weist sie eine Wärmequelle auf. Des weiteren weist sie einen Erzeuger für Vibrationen auf, der in der Vorrichtung oder in der Fernbedienung/Fernanzeige angeordnet ist.

## Patentansprüche

1. Vorrichtung (1) in Kissenform für gymnastische Zwecke, auf die vom Menschen eine Druckkraft ausübbar ist, **gekennzeichnet** durch eine Einrichtung zum Messen und/oder Anzeigen des auf die Vorrichtung durch den menschlichen Körper ausgeübten Druckes, wobei die Mess- und/oder Anzeigeeinrichtung bei Überschreiten eines bestimmten Druckes ein akustisches und/oder optisches Signal abgibt.

2. Vorrichtung nach Anspruch 1, **dadurch gekennzeichnet,** daß während des Einhaltens des bestimmten Druckes und/oder nach dem Einhalten des bestimmten Druckes für eine bestimmte vorwählbare Dauer das akustische und/oder optische Signal abgegeben wird.

3. Vorrichtung nach einem der vorherigen Ansprüche, **dadurch gekennzeichnet,** daß die gesamte Unterseite (5) eben ist.

4. Vorrichtung nach einem der vorherigen Ansprüche, **dadurch gekennzeichnet,** daß die Oberseite (3) im Längs- und/oder Querschnitt gewölbt ist und die Wölbung bogenförmig, kreisabschnittförmig, ellipsenabschnittförmig oder korbbogenförmig ist.

5. Vorrichtung nach einem der vorherigen Ansprüche, **dadurch gekennzeichnet,** daß sie eine größere Länge als Breite aufweist.

6. Vorrichtung nach einem der vorherigen Ansprüche, **dadurch gekennzeichnet,** daß die größte Höhe (H) gleich oder kleiner als 5 cm insbesondere 1 bis 3 cm ist.

7. Vorrichtung nach einem der vorherigen Ansprüche, **dadurch gekennzeichnet,** daß die Außenseite von einem Schlauch gebildet ist.

8. Vorrichtung nach einem der vorherigen Ansprüche, **dadurch gekennzeichnet,** daß sie über eine Leitung und/oder Schlauch mit einer Fernbedienung (8) und/oder eine Fernanzeige (8) verbunden ist.

9. Vorrichtung nach einem der vorherigen Ansprüche, **dadurch gekennzeichnet,** daß sie eine Wärmequelle aufweist.

10. Vorrichtung nach einem der vorherigen Ansprüche, **dadurch gekennzeichnet,** daß sie einen Erzeuger für Vibrationen aufweist, der in der Vorrichtung oder in der Fernbedienung/Fernanzeige angeordnet ist.
